Europäisches Patentamt

European Patent Office

Office européen des brevets

(19)

(11) Publication number: **0 070 675**
**A1**

(12) **EUROPEAN PATENT APPLICATION**

(21) Application number: 82303667.8

(22) Date of filing: **13.07.82**

(51) Int. Cl.³: **C 12 N 15/00**, C 12 P 21/02, C 07 C 103/52

(30) Priority: 15.07.81 GB 8121699
26.11.81 GB 8135738

(43) Date of publication of application: 26.01.83
Bulletin 83/4

(84) Designated Contracting States: **CH DE FR GB IT LI NL SE**

(71) Applicant: **Craig, Roger Kingdon, The Courtauld Institute of Biochemistry The Middlesex Hospital School, London W1P 7PN (GB)**
Applicant: **MacIntyre, Iain, The Royal Postgraduate Medical School Hammersmith Hospital, London W12 0HS (GB)**

(72) Inventor: **Craig, Roger Kingdon, The Courtauld Institute of Biochemistry The Middlesex Hospital School, London W1P 7PN (GB)**
Inventor: **MacIntyre, Iain, The Royal Postgraduate Medical School Hammersmith Hospital, London W12 0HS (GB)**

(74) Representative: **Votier, Sidney David et al, CARPMAELS & RANSFORD 43, Bloomsbury Square, London WC1A 2RA (GB)**

(54) **Human calcitonin precursor polyprotein structural gene.**

(57) There is described the construction of recombinant plasmids containing human calcitonin cDNA sequences, using total poly(A)-containing RNA isolated from a human medullary carcinoma of the thyroid, the identification and characterisation thereof, and the use of such plasmids in the production of human calcitonin.

EP 0 070 675 A1

"HUMAN CALCITONIN PRECURSOR POLYPROTEIN STRUCTURAL GENE"

This invention relates to the field of recombinant DNA biotechnology. In particular it relates to the use of recombinant DNA biotechnology in the production of human calcitonin precursor structural gene, the insertion of said gene in a vector system, the cloning thereof and subsequently the production of human calcitonin.

Calcitonin is a small polypeptide hormone consisting of 32 amino acid residues (3500 mol.wt) synthesized and secreted in humans by the C-cells of the thyroid gland.

The main physiological function of calcitonin is to limit skeletal breakdown during times of calcium and phosphorus requirement, for example during growth, pregnancy and lactation. Thus for instance the absence of calcitonin during rapid growth in adolescence causes bone loss. A similar loss of bone may occur in pregnancy, when calcitonin secretion is absent. Calcitonin levels in men are higher than in women, and in both sexes, levels decline with age. This is particularly apparent in women after the menopause, where low levels of calcitonin appear to be an important factor in post-menopausal bone loss and osteoporosis. It is probable that should human calcitonin become commercially available in sufficient quantity, the peptide will be used alone or in combination with other drugs in the prevention and treatment of post-menopausal osteoporosis. Calcitonin may also be helpful in the treatment of elevated plasma calcium due to malignant deposits in bone. The commonest cause of this is cancer in the breast with secondary deposits in the skeleton. The action here probably reflects in part inhibitory action of calcitonin in osteoclasts. The latter are partly responsible for increased bone destruction leading in turn to elevated plasma calcium levels. Calcitonin may also have a direct action on cancer cells in bone and thus may play a role in the action of calcitonin in lowering elevated plasma calcium levels due to malignant deposits in bone.

0070675

At present, calcitonin (purified from salmon) is used in pharmacological rather than replacement doses, for the treatment of Paget's disease.   This is a common condition affecting, in the main, people over 40 years of age.   In this group as many as 4% of the population may be affected.

Thus in the U.K., for instance, although the disease is usually asymptomatic, there are tens of thousands of people needing treatment. Unfortunately prolonged treatment with salmon calcitonin has proved impracticable due to immuno-
in about 25% of cases
logical rejection of the fish calcitonin variant/. Consequently there is already a need for large amounts of human calcitonin.

We have elucidated the fine structure of a human calcitonin structural gene, which may be inserted into a prokaryote or eukaryote cell system by known recombinant DNA techniques, such that human calcitonin, in addition to other peptides encoded within the structural gene, may be synthesised in large quantities, purified, and subsequently used for pharmacological purposes.

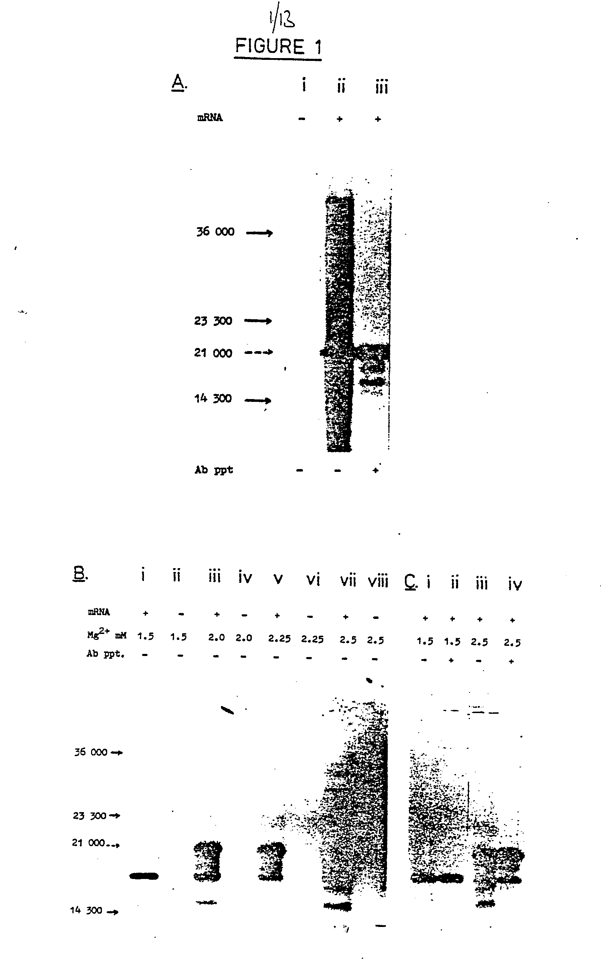

Total human poly(A)-containing mRNA isolated from a medullary carcinoma of the thyroid directs the synthesis in cell-free protein synthesizing systems of a major polypeptide of estimated mol.wt. 21000. This, and a series of minor polypeptides of lesser abundance are precipitable with antiserum raised against synthetic human calcitonin. The experiments conducted to show this are described
(shown in Figure 1)
hereinafter and the results/contrast markedly with the known mol.wt. of calcitonin circulating in normal human serum (3500).

Analysis of the mRNA species which direct the synthesis of these high molecular weight presumptive calcitonin precursors, was carried out by a combination of recombinant DNA technology, DNA sequence analysis, and size determination of the calcitonin mRNA. Thus total poly(A)-containing RNA known to direct the synthesis of high molecular weight immunoprecipitable forms of human calcitonin, was used as a template to synthesize a double-stranded cDNA population. This was inserted into plasmid DNA using established procedures and transformed into a suitable E.coli host. Colonies containing cDNA sequences representative of the most abundant thyroid poly(A)-containing RNA populations were selected using in situ hybridisation procedures, and those containing

calcitonin cDNA sequence then identified by hybridisation translation. We also describe here the detailed analysis of two of these plasmids designated phT-B3 and phT-B6, and their subsequent use to determine the size, sequence and coding potential of those mRNA(s) encoded by the calcitonin structural gene(s).

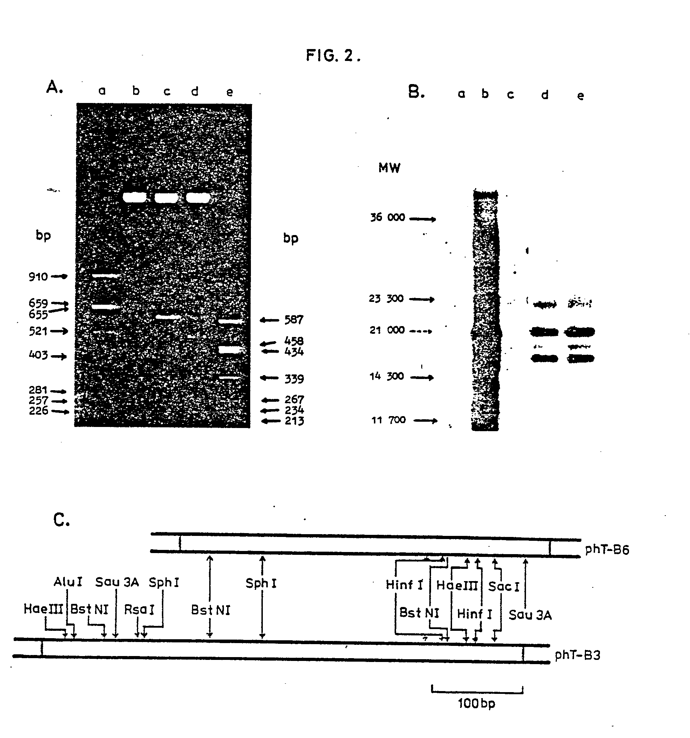

The results depicted in Figure 2 demonstrate:-

(A)  That the recombinant plasmids contain additional DNA sequence (phT-B6, 490 bps; phT-B3, 590 bps) when compared with the parental plasmid DNA, as determined by restriction analysis using the endonuclease Pst I.

(B)  That both recombinant plasmids contain sequences capable of hybridising a thyroid mRNA species which directs the synthesis of the presumptive human calcitonin precursor polyprotein in a cell-free protein synthesizing system.

(C)  That both recombinant plasmids contain cDNA sequence in common as judged by restriction endonuclease mapping, but that phT-B3 contains additional sequence at one end of a common fragment, and phT-B6 additional sequence at the other.

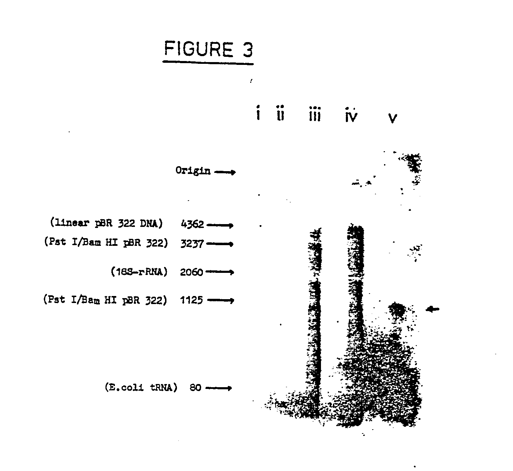

Figure 3 shows the results of an RNA blotting experiment using a $^{32}$P-labelled phT-B3 hybridisation probe. From this it is apparent that the mRNA species in question are present in large amounts in thyroid tissue and are $1000 \pm 100$ nucleotides in length.

That data described above defines the source of calcitonin mRNA, the size of the mRNA and demonstrates that calcitonin is synthesized as a high molecular weight precursor polyprotein which in vivo must require extensive post-translational processing prior to secretion.

DNA sequence analysis of the cloned cDNA sequences

(i)  defines the relative position of the calcitonin peptide within this precursor polyprotein.

(ii)  reveals the amino acid sequence of the flanking $NH_2$-terminal and COOH-terminal peptides.

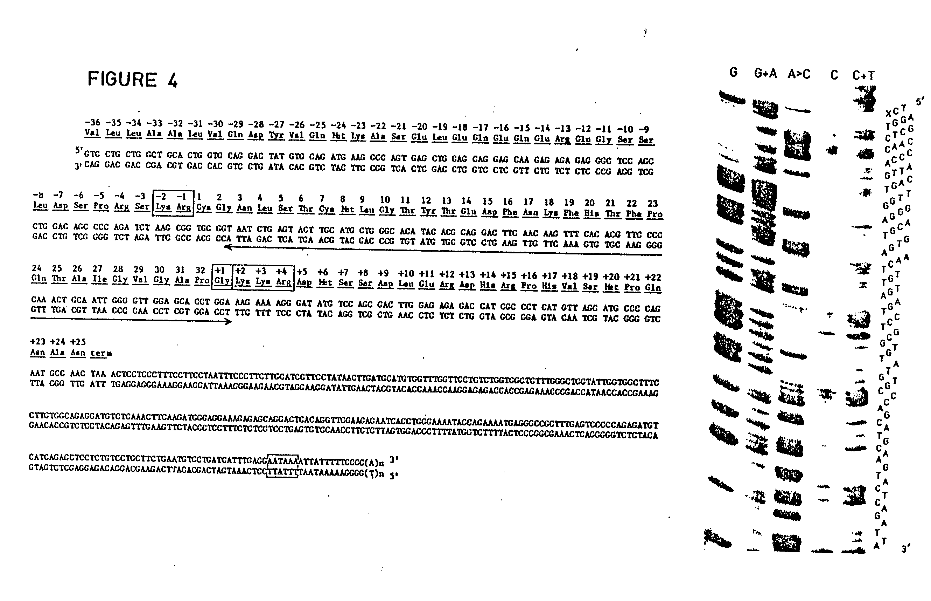

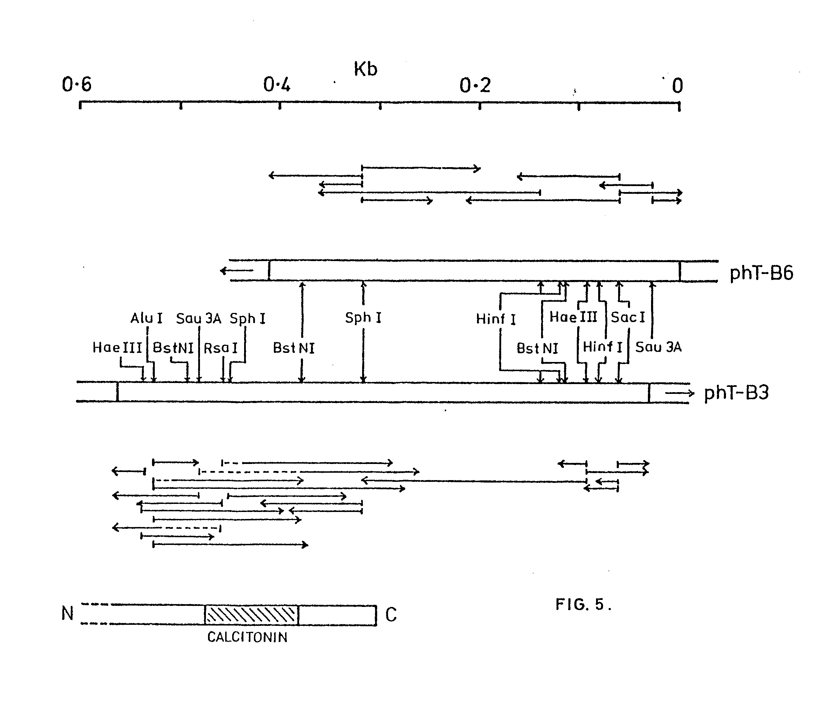

Figure 4 depicts the nucleotide sequence of cDNA inserted into plasmids phT-B3 and phT-B6 and Figure 5 depicts the DNA sequencing strategy employed to determine this sequence. One (phT-B6) contains the whole of the 3' untranslated region of the mRNA, part of the sequence encoding the calcitonin peptide, and reveals that the mRNA encodes a further 25 amino acids after the COOH-terminal proline of calcitonin before a stop codon is encountered in phase. The other (phT-B3) contains a sequence which specifies the whole of the calcitonin peptide, the additional 25 amino acids of the flanking COOH-terminal (cryptic) peptide, a 36 amino acid $NH_2$-terminal (cryptic) flanking peptide, and most of the 3' untranslated region of the mRNA. In all we have sequenced 580 bases or 60% of the total human calcitonin mRNA using two overlapping cDNA clones.

The human calcitonin structural gene(s) encode mRNA species of $1000^{\pm}100$ bases in length. Translation of these mRNAs gives rise to a 21,000 mol.wt. precursor polyprotein. The calcitonin peptide resides towards the COOH-terminus of this polyprotein, flanked on the COOH-terminal side by an additional 25 amino acids, and on the $NH_2$-terminal side by an amino-acid sequence of as yet undetermined length though greater than 36 amino acids in length.

Thus, in one aspect the present invention comprises human calcitonin precursor polyprotein structural gene.

The invention also comprises a DNA transfer vector, especially a plasmid, having an inserted polypeptide fragment including the following amino acid sequence:-

-cys-gly-asn-leu-ser-thr-cys-met-leu-gly-thr-
-tyr-thr-gln-asp-phe-asn-lys-phe-his-thr-phe-
-pro-gln-thr-ala-ile-gly-val-gly-ala-pro.

The invention also includes DNA transfer vector, especially a plasmid, having an inserted polypeptide fragment which includes the following amino acid sequence:-

-val-leu-leu-ala-ala-leu-val-gln-asp-tyr-val-gln-met-
-lys-ala-ser-glu-leu-glu-gln-glu-gln-glu-arg-glu-gly-
-ser-ser-leu-asp-ser-pro-arg-ser-lys-arg-
-cys-gly-asn-leu-ser-thr-cys-met-leu-gly-thr-

0070675

-tyr-thr-gln-asp-phe-asn-lys-phe-his-thr-phe-
-pro-gln-thr-ala-ile-gly-val-gly-ala-pro-
-gly-lys-lys-arg-asp-met-ser-ser-asp-leu-glu-arg-
-asp-his-arg-pro-his-val-ser-met-pro-gln-asn-ala-asn-.

Further according to the invention a method of forming a DNA transfer vector having a nucleotide sequence coding for a polypeptide comprising the amino acid sequence of human calcitonin comprises

(i)   providing mRNA coding for a polypeptide comprising the amino acid sequence of human calcitonin,

(ii)   synthesizing a double stranded cDNA one strand of which has a nucleotide sequence complementary to that of the mRNA, and

(iii) inserting said double stranded cDNA in a DNA transfer vector.

The mRNA is suitably provided by cells containing the mRNA originating from the thyroid gland of a calcitonin-producing organism.  Other sources of such cells include the lung and brain.   ....

The DNA transfer vector is suitably transferred to and replicated in a microorganism strain, e.g. a bacterium such as Escherichia coli.

The invention also provides a polypeptide containing the amino acid sequence of human calcitonin which is processable, suitably by cleaving, to produce human calcitonin.  Such polypeptide is suitably a fusion protein comprising a host protein in combination with a peptide comprising the amino acid sequence of human calcitonin.

The invention still further provides a method for the production of human calcitonin comprising processing a polypeptide to produce human calcitonin in which said polypeptide has been expressed by a host organism which has been transformed with a gene-containing DNA transfer vector comprising an inserted gene encoding a polypeptide comprising the amino acid sequence of human calcitonin.

There follows a description of experimental work undertaken to construct and characterise plasmids containing complementary DNA sequences to human calcitonin precursor polyprotein, and the subsequent production of human calcitonin from trp E-calcitonin fusion proteins.

I.   Materials and Methods

a.   Materials.

Restriction enzyme PstI was obtained from Boehringer and calf thymus terminal deoxynucleotidyl transferase from P.L.Biochemicals.  All other enyzymes were obtained from sources described previously (Craig R.K.Hall, L.Parker, D. & Campbell, P.N. (1981) Biochem.J.194, 989-998).  L-[$^{35}$S]-methionine (700-1300Ci/mmol), deoxy[5-$^{3}$H]cytidine 5'-triphosphate (18.4Ci/mmol), deoxy[8-$^{3}$H]guanosine 5'-triphosphate (11.7Ci/mmol), adenosine 5'-[$\gamma$-$^{32}$P]triphosphate (2000-3000Ci/mmol) and deoxyguanosine 5'-[$\alpha$-$^{32}$P]triphosphate ($\geqslant$400Ci/mmol) were from  Amersham International; AMV reverse transcriptase (lot no.G-91180) was provided by Dr.J.W.Beard, Life Sciences Inc., St.Petersberg, FL 33707, U.S.A.   All other chemicals and solvents were obtained from sources previously described (Craig, R.K.Brown, P.A., Harrison O.S.McIlreavy, D.& Campbell, P.N. (1976)Biochem, J.160, 57-74 Craig, R.K.Boulton, A.P.Harrison, O.S.Parker, D.& Campbell, P.N. (1979)Biochem.J.181, 737-756); (Pascall, J.C.Boulton, A.P.Parker, D.,Hall, L.& Craig, R.K. (1981) Biochem.J.196, 567-574).

b.   Isolation of poly(A)-containing RNA from human medullary carcinoma of the thyroid: the construction, transformation and selection of recombinant plasmids.

Total RNA was isolated from frozen human thyroid medullary carcinoma tissue as described by (Hall,L.Craig, R.K.& Campbell, P.N. (1979) Nature (London) 277,54-56), and residual DNA removed by digestion with deoxyribonuclease as described by Zimmerman, S.B. and Sandeen G.(1966) Anal.Biochem.14, 269-277.  Poly (A)-containing RNA was then isolated by affinity chromatography on oligo-(dT)-cellulose, and a size-selected double-stranded cDNA population was then synthesized from the resulting poly(A)-containing RNA population as described previously (Craig et al., 1981; Hall,L.Davies, M.S. & Craig, R.K. (1981) Nucleic Acids Res.9, 65-84). The size-selected double-stranded cDNA was then extended at the 3'-hydroxyl termini with dC residues in the following manner. Double-stranded cDNA ($1\mu g$/ml was dissolved in 30mM-Tris/100mM-cacodylic acid (adjusted to pH7.5 with 10M-KOH) containing 2mM-$CoCl_2$, 0.1mM-dithiothreitol, $50\mu g$ of nuclease-free bovine serum albumin/ml, and 0.1mM-$[^3H]$ dCTP (4Ci/mmol). Terminal deoxynucleotidyl transferase (300units/ml) was then added and the mixture incubated at 30°C. When a 30-50 residue long poly(dC)tail had been added (approx. 15min as determined by trichloroacetic acid precipitation of $1\mu l$ amounts at 5min intervals), the reaction was/terminated by extraction with phenol/chloroform, and the nucleic acid was recovered by ethanol precipitation. The precipitate was washed twice with ethanol, dissolved in double-distilled water and stored at -70°C.

Highly purified supercoiled pAT153 DNA prepared as described previously (Craig et al., 1981) was digested with the restriction endonuclease PstI, then tailed with poly(dG)$_{12}$ essentially as described above, except that the DNA was present at $50\mu g$/ml. $[^3H]$dGTP (1.7Ci/mmol) replaced dCTP as the radiolabelled deoxynucleotide triphosphate, and terminal deoxynucleotidyl transferase was present at a final concentration of 800units/ml. Poly-(dC)-tailed cDNA and poly(dG)-tailed pAT153 DNA were then annealed in 10mM-Tris/HCl, pH7.6, containing 200mM-NaCl and 1mM-EDTA, in polypropylene 0.4ml "snap-cap" tubes at a final concentration of $0.4\mu g$/ml and $4\mu g$/ml respectively. The mixture was heated in a water bath to 70°C for 30min, the water bath switched off, and the hybridization mixture allowed to cool to room temperature overnight. The amount of poly(dC)-tailed cDNA used for transformation varied from 10 to 20 ng.

The resulting chimaeric plasmid DNA was then used to transform <u>Escherichia</u> <u>coli</u> HB101 HB101 rec A⁻ (Boyer, H.W. & Roulland-Dussoix, D. (1969) J.Mol.Biol. 41, 459-472), under conditions of good microbiological practice in accordance with the guidlines laid down by the British Genetic Manipulation Advisory Group, using methodology described elsewhere (Craig et al., 1981). Transformed cells were selected on 1.5% (w/v) agar in L-broth containing 12.5µg of tetracycline/ml. Individual colonies were then replica plated onto fresh agar plates containing 12.5µg of tetracycline/ml alone or 100µg of ampicillin/ml alone. Those colonies found to be sensitive to ampicillin were selected for subsequent experimentation.

c.  <u>Identification of plasmids containing human calcitonin precursor polyprotein cDNA sequences, and characterization of calcitonin precursor polyprotein mRNA.</u>

Colony filter hybridization in situ, plasmid growth and purification, positive hybridization-translation, purification of individual cDNA sequences, and size estimation of individual mRNA sequences using the "Northern" transfer technique have been described elsewhere (see Craig et al., 1981; Burditt, L.J.Parker, D.Craig, R.K. Getova, T. & Campbell, P.N.(1981)Biochem, J. 194 999-1006; Hall et al., 1981). Nick-translation of plasmids was as decribed by Rigby, P.W.J.Dieckmann, M.Rhodes, C.& Berg. P. (1977) J.Mol.Biol.113, 237-251). Unless specifically stated otherwise all restriction enzyme analyses were performed using the ionic conditions recommended by the manufacturers. Size analysis of restricted plasmid DNA was performed by electrophoresis on flat-bed agarose gels as described elsewhere (Craig et al., 1981).

d.  <u>Cell-free protein synthesis and product analysis.</u>

mRNA-directed cell-free protein synthesis in the wheat-germ cell-free protein synthesizing system and antibody precipitation procedures were as described by Craig et al., (1976). SDS/polyacrylamide-gel electrophoretic analysis was performed using slab gels as described by Pascall et al. (1981). Fluorography followed the procedure of (Bonner, W.M. & Laskey, R.A.(1974) Eur.J.Biochem. 46, 83-88) except that dimethylsulphoxide was replaced by glacial acetic acid (Burckhardt, J.Telford, J. & Birnstiel, M.L. (1979) Nucleic Acids Res. 6, 2963-2971).

II.  Results

e.  Isolation and characterization of a poly(A)-containing RNA population from human medullary thyroid carcinoma tissue.

Total human poly(A)-containing RNA isolated from a medullary carcinoma of the thyroid was added to a wheat-germ cell-free protein synthesizing system, and the resulting $[^{35}S]$methionine-labelled proteins were analysed by SDS/polyacrylamide-gel electrophoresis and fluorography. The results (Fig.1a) demonstrate that a spectrum of proteins up to mol.wt.60000 was synthesized. The most prominent of these, a protein of estimated mol.wt.21000, proved to be precipitable with antiserum raised against human calcitonin. In addition to this protein, three less abundant peptides of estimated mol.wt.19500, 18000 and 15000 were also precipitated by calcitonin-specific antiserum. It is believed that these may represent partially processed calcitonin precursor polyproteins, a result of post-translational processing activities present in the wheat-germ cell-free extract (see Pascall et al. 1981). Alternatively these may result from the translation of multiple poly(A)-containing species encoding different polyproteins each containing the calcitonin amino acid sequence. These differences were particularly apparent at $Mg^{2+}$ concentrations which were suboptimal for protein synthesis. Thus at $1.5mM-Mg^{2+}$ the predominant polypeptide was the mol.wt. 18000 calcitonin precursor polyprotein form whilst at $2.5mM-Mg^{2+}$, the optimum for protein synthesis, the mol.wt. 21000 calcitonin precursor polyprotein form predominant (see Fig.1b). Immaterial of the $Mg^{2+}$ concentration, the predominant polypeptides remained precipitable with antiserum raised against human calcitonin (Fig.1c).

Overall, on the basis of the sources of tissue and the identification of polypeptide immunoprecipitable with human calcitonin antisera as the predominant products of mRNA-directed cell-free protein synthesis, we conclude that an abundant poly(A)-containing RNA species present in human thyroid medullary carcinoma tissue directs the synthesis of calcitonin precursor polyprotein(s).

f.    Construction of recombinant cDNA plasmids using total poly(A)-containing RNA isolated from a human medullary carcinoma of the thyroid.

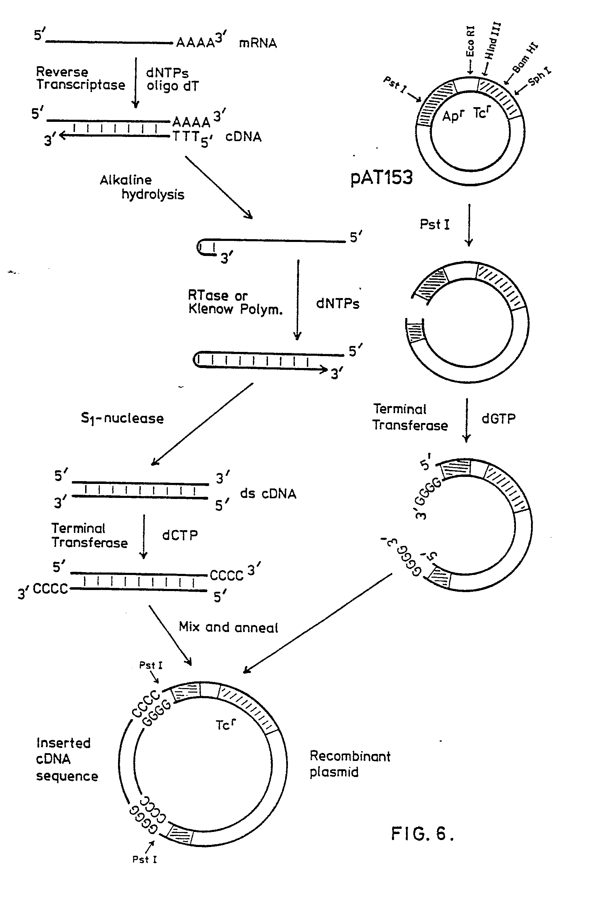

All manipulations followed the procedures outlined for the construction of recombinant plasmids containing human and guinea-pig milk protein cDNA sequences (see Craig et al., 1981; Hall et al., 1981) except where specifically stated in the Methods section using the overall strategy outlined in Fig.6.

Briefly, cDNA representative of the total poly(A)-containing RNA population isolated from the human medullary carcinoma of the thyroid was synthesized by using AMV reverse transcriptase. Size analysis using agarose-gel electrophoresis showed that the bulk of the cDNA synthesized ranged in size from 500 to 1500 nucleotides in length. This was converted to a double-stranded form using AMV reverse transcriptase, followed by $S_1$ nuclease excision of the resultant hairpin loop. This population was then sized by preparative agarose-gel electrophoresis and those sequences ranging from 300 to 2500 nucleotides in length were eluted from the gel, and their 3'-hydroxyl termini extended with poly(dC)$_{50}$ using calf thymus terminal deoxynucleotidyl transferase. This population was then annealed with PstI-restricted pAT153 DNA which had previously been extended at the 3'-hydroxyl termini with poly(dG)$_{12}$. The resulting chimaeric molecules were used to tranform a rec A$^-$ strain of E.coli HB101 under conditions of good microbiological practice. Transformants were selected on L-agar plates containing tetracycline, and colonies containing recombinant plasmids were then selected by replica plating onto L-agar containing ampicillin alone, or tetracycline alone.  In total a
single transformation using 20ng of double-stranded tailed
cDNA yielded 107 colonies that had lost sensitivity to
ampicillin, and therefore were presumed to contain cDNA
sequences inserted at the PstI site of pAT153.

g.    Identification and characterization of recombinant
      plasmids containing human calcitonin cDNA sequences.

It was apparent from cell-free protein synthesizing
studies that the human medullary carcinoma of the thyroid
poly(A)-containing RNA population contained an abundant
mRNA population which directed the synthesis of calcitonin
precursor polyprotein(s).  Consequently total base-cleaved
$^{32}$P-labelled poly(A)-containing RNA from human medullary
carcinoma was used to identify, in a preliminary manner
using in situ hybridization procedures, those colonies
most likely to contain calcitonin precursor  polyprotein
cDNA sequences (see the Methods section).  All the am-
pillin-sensitive colonies were screened in this manner,
and 17 (16%) showed significant hybridization over the
background.  Of these, 11 were selected and grown in
liquid culture, the plasmid DNA was isolated, and the
presence of additional DNA sequence determined by digestion
with the restriction endonuclease PstI, followed by
agarose-gel electrophoresis.  This confirmed that all
but one contained additional DNA as compared with the
parental plasmid pA153.  The detailed characterization
of two plasmids, phT-B3 and phT-B6, is described.

Size analysis of the inserted cDNA sequences by
agarose-gel electrophoresis after excision of the cDNA
by digestion with the restriction endonuclease PstI,
showed (Fig.2A) that phT-B3 and phT-B6 contained 590 and
490 base pairs of inserted sequence.

Identification of the coding sequences present in
each plasmid was carried out by SDS/polyacrylamide-gel
electrophoresis of the wheat-germ cell-free translation
products of mRNA isolated by hybridization under stringent conditions to partially restricted denatured plasmid
DNA immobilized on DBM-paper filters (see Craig et al.,

1981). This demonstrated that both plasmids contained DNA sequences complementary to the medullary thyroid carcinoma mRNA species which directed the synthesis of the abundant calcitonin precursor protein (Fig.2B). In each instance, although cell-free protein synthesis was performed in the presence of $2.5mM-Mg^{2+}$, both the 21000 and 18000 molecular weight calcitonin precursor polyprotein forms were present. Subsequent mapping of these sequences using a variety of restriction endonucleases (Fig.2C) showed that both recombinant plasmids contained cDNA sequence in common, but that phT-B3 contained an additional sequence at one end of the common fragment and phT-B6 an additional sequence at the other.

To determine the proportion of the calcitonin precursor polyprotein mRNA sequence within the characterized recombinants, the size of the mRNA was estimated using RNA blotting techniques (Alwine, J.C.Kemp, D.J.& Stark, G.R. (1977) Proc. Natl.Acad.Sci.U.S.A. 74, 5350-5354) after separation of glyoxal-treated poly(A)-containing RNA (McMaster, G.K.& Carmichael, G.G. (1977) Proc.Natl.Acad. Sci.U.S.A. 74, 4835-4838) by horizontal agarose-gel electrophoresis (Fig.3). This demonstrated that the human calcitonin precursor polyprotein mRNA was $1000\pm100$ nucleotides in length. Thus the cDNA sequence inserted into phT-B3 and phT-B6 represented in total 60-65% of the mRNA sequence. A parallel analysis of the non-polyadenylated human thyroid medullary carcinoma RNA also revealed the presence of calcitonin precursor polyprotein mRNA of identical length, but also discrete bands of lower molecular weight, either partially degraded mRNA or discrete lower molecular weight mRNA species containing sequence in common with the defined calcitonin sequence (see Fig.4). Calcitonin precursor polyprotein mRNA was not present in detectable amounts, under the conditions employed, in total poly(A)-containing RNA isolated from full-term human placental tissue, or from T-47D cells, a human cell line of mammary origin (see Fig.3).

Explanation of Figures 1 to 6 of the drawings.

Figure 1.mRNA directed cell-free protein synthesis of human calcitonin precursor polyproteins as judged by immuno-precipitation and SDS/polyacrylamide-gel electrophoresis.

Total poly(A)-containing RNA (0.2µg/assay) isolated from frozen human thyroid medullary carcinoma tissue (4g) (see Hall et al., (1979) Nature (London) 277, 54-56) was added to a wheat-germ cell-free protein-synthesizing system and the resulting [$^{35}$S]methionine-labelled proteins were separated by using SDS/polyacrylamide-gel electrophoresis, then visualized using fluorography (see Materials and methods above). (A) Lane (i), no added mRNA; lane (ii), human thyroid medullary carcinoma poly(A)-containing RNA; lane (iii), as in lane (ii) but proteins were precipitated using antiserum raised against synthetic human calcitonin. (B) Lanes (i),(iii),(v) and (vii), human thyroid medullary carcinoma poly(A)-containing RNA translated at Mg$^{2+}$ concns. of 1.5,2.0,2.25 and 2.5mM respectively. Lanes (ii),(iv), (vi) and (viii), no added RNA at Mg$^{2+}$ concns. of 1.5,2.0, 2.25 and 2.5mM respectively. (C) Lanes (i) and (iii), human thyroid medullary carcinoma poly(A)-containing RNA translated at Mg$^{2+}$ concns. of 1.5 and 2.5mM respectively; lanes (ii) and (iv), as in lanes (i) and (iii) respectively, but proteins precipitated using antiserum raised against synthetic human calcitonin. Arrows represent the relative mobility of the following Coomassie Blue stained marker proteins of known molecular weight, (see Weber et al., 1972); glyceraldehyde-3-phosphate dehydrogenase (36000); tryspin (23300); and lysozyme (14309).

Figure 2. Characterization of plasmids containing human calcitonin precursor polyprotein cDNA sequences. (A) Size analysis of inserted cDNA sequences. Plasmid DNA samples (0.25µg) were digested with restriction endonucleases, then electrophoresed on a 1.6% (w/v) agarose-gel as described previously (see Hall et al., 1981). DNA fragments of known size (see Sutcliffe, J.G.(978a) Cold Spring Harb. Symp. Quant.Biol. 43, 77-90; Sutcliffe, J.G. (1978b) Nucleic Acids Res. 5, 2721-2728) were generated by AluI digestion of pBR322 DNA and HaeIII digestion of pAT153 DNA.Lane (a), AluI markers of 910,659,655,521,403,281,257 and 226 base pairs; lane (b), pAT153 DNA digested with PstI; lane (c), phT-B3 DNA digested with PstI; lane (d) phT-B6 DNA digested with PstI; lane (e), HaeIII markers of 587,458,434,339,267, 234 and 213 base pairs. (B) Positive hybridization translation. Sequence-specific poly(A)-containing RNA isolated by hybridization to partially restricted, denatured recombinant DNA immodilized on DBM-paper was added to a wheat-germ cell-free system and the [$^{35}$S]methionine-labelled cell-free translation products were separated by SDS/polyacrylamide-gel electrophoresis, then visualized by fluorography. Lane (a), no added RNA; lane (b), total human thyroid medullary carcinoma poly(A)-containing RNA; lanes (c), (d) and (e), poly(A)-containing RNA isolated by hybridization to plasmids pAT153, phT-B3 and phT-B6 respectively. Arrows denote the relative position of marker proteins as described in relation to Fig.1. but in addition include cytochrome-C (11700). (c) Comparative restriction endonuclease maps of the inserted cDNA sequence within recombinant plasmids phT-B3 and phT-B6.

Figure 3. Size estimation of human calcitonin precursor polyprotein mRNA.

RNA samples were treated with glyoxal, then loaded onto separate slots on a 1.5% (w/v) agarose-gel, electrophoresed and then blotted onto DBM-paper as described previously (Craig et al., 1981). The position of the human calcitonin precursor polyprotein mRNA sequence (arrowed) was determined by hybridization to a $^{32}$P-labelled nick-translated phT-B3 plasmid DNA hybridization probe (5.6 x $10^7$ c.p.m./$\mu$g). Lane (i), T-47D poly(A)-containing RNA (1$\mu$g); lane (ii), human placental poly(A)-containing RNA (1$\mu$g); lane (iii), total human thyroid medullary carcinoma RNA (40$\mu$g); lane (iv), non-polyadenylated human thyroid medullary carcinoma RNA (40$\mu$g); lane (v), human thyroid medullary carcinoma total poly(A)-containing RNA (1$\mu$g).

Size estimations were determined by direct comparisons
with the mobilities of glyoxal-treated linear pBR322 DNA
(4362 bases), rabbit reticulocyte 18S RNA (2060 bases), a
BamHI/PstI digest of pBR322 DNA (3237 and 1125 bases),
and E.coli tRNA (80 bases).

As described above, Figure 4 shows the nucleotide
sequence of the human calcitonin precursor polyprotein
structural gene and the amino acids it encodes. The amino
acids labelled 1-32 correspond to the previously known
amino acid sequence of human calcitonin.

The amino acid sequence -1 to -36 represents the
previously unknown cryptic $NH_2$-terminal flanking peptide,
and the amino acid sequence +1 to +25 the cryptic carboxyl-
terminal flanking peptide. Arrows define the region of
nucleotide sequence which may be deduced from the adjacent
DNA sequencing ladder. Boxed amino acids (-2 and -1;
+1 to +4) represent predicted sites for proteolytic processing in vivo (see Steiner D.F.,Quinn, P.S. Chan.S.J.
Marsh J. and Tager H.S. (1980) Ann. N.Y. Acad.Sci. 343,
1-16). The glycine resdue (+1), an important consideration
in the construction of the expression vectors described
below, is in vivo required during processing events for the
amidation of the adjacent carboxyl terminal amino acids
(Kreil, G., Suchanek, G. and Kindas-Mugge, I. (1977) Fedn.
Proc. 36, 2081-2086). Thus in human calcitonin glycine
+1 is required for the amidation of the carboxyl-terminal
proline residue (32), a feature required for biological
activity.

Figure 5 defines the nucleotide sequencing strategy
carried out using the chemical cleavage procedure of
Maxam A.M. and Gilbert W.Procn. Natn. Acad. Sci. U.S.A. 74
560-564 (1977).

Figure 6 depicts the overall cloning strategy.

There now follows a description of the production,
using the expression vector designated pCT37, the combination

therewith of the human calcitonin sequence derived from plasmid phT-B3 to produce a fusion protein, and the production therefrom of either human calcitonin or human calcitonin with an additional glycine residue at the carboxy-terminal end.

ISOLATION OF THE TRYPTOPHAN PROMOTER AND TRP E GENE

The tryptophan operon has been the subject of much attention in recent years. The result of this is the elucidation of the complete nucleotide sequence of the five structural genes and their control regions ( C Yanofsky et al  Nucleic Acids Res  9, 6647-6668, 1981). The control regions and parts of the structural genes have also been used to construct vectors for the expression of foreign genes (B E Enger-Valk et al Gene 9, 69-85, 1980; W Tacon, N Carey and J S Emtage, Molec Gen Genet 177, 427-438, 1980; R A Hallewell and J S Emtage, Gene 9, 27-47, 1980). Earlier work had also shown that the control regions and the complete trp E gene could be isolated from the E coli chromosome on a Hind III fragment of ≅ 5700 bp (A S Hopkins, N E Murray and W J Brammar  J Mol Biol 107, 549-569, 1976). The E coli operon has also been isolated from the DNA of trp transducing strains of phages ∅ 80 and λ (B E Enger-Valk et al loc cit).

Alternatively, the control region and trp E gene  can be isolated using cosmid vectors and in vitro packaging (B Hohn and K murray Proc  Natl Acad Sci  USA 74, 3259-3262, 1977; Collins, J and Hohn, B Proc Natl Acad Sci  USA 75, 4242-4246, 1978). Thus, E coli chromosomal DNA was partially digested with Sau 3A and the resulting fragments ligated to the 10.3 kb cosmid 3030 that had previously been digested to completion with Bam HI. The cosmid 3030 contains a unique site for Bam HI and confers resistance to ampicillin. After ligation, the mixture was packaged in vitro and used to infect a trp E⁻ strain of E coli K12. Recombinants were selected on L-agar plates containing ampicillin from which they were replica plated onto M9 salts, glucose, casamino acids minimal agar plates supplemented with ampicillin. Recombinants were thus selected that complemented the trp $E^-$ strain by their ability to grow in the absence of tryptophan. Several recombinants were identified and plasmid DNA isolated and characterised by restriction enzyme analysis. One cosmid, designated 3030/trp, producing a 5700 bp fragment on digestion with Hind III was selected for further use.

Cosmid 3030/trp was digested with Hind III, the fragments separated by agarose gel electrophoresis and the 5700 bp fragment recovered from a gel slice. Finally, this 5700 bp fragment was cloned into the Hind III site of pAT153 to produce the plasmid ptrp $E_{5700}$.

CONSTRUCTION OF pCT12

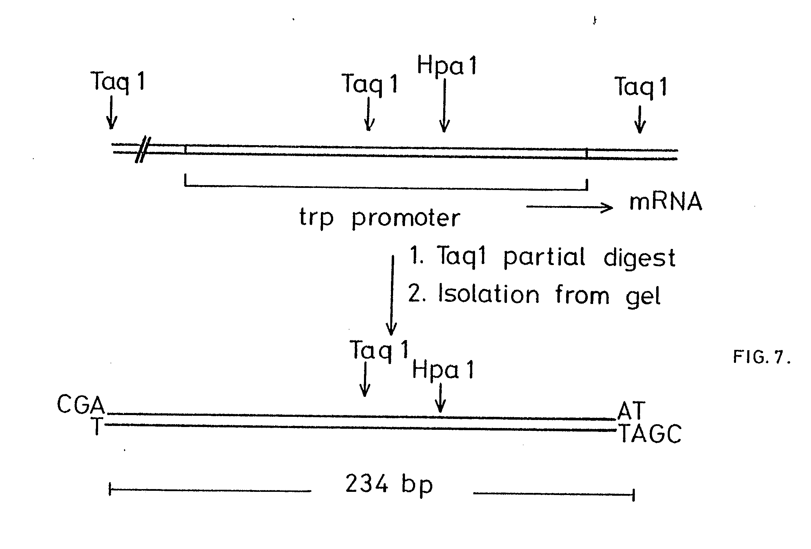

A schematic representation of a portion of the tryptophan promoter-containing fragment is given in Fig 7. From this figure it can be seen that the complete tryptophan promoter-operator complex can be isolated on a Taq I restriction fragment of 234 base pairs (bp) if the DNA is digested under conditions where about 50% of the available sites are cleaved. Thus, 10 µg of ptrp $E_{5700}$ was incubated in 10 mM Tris-HCl pH 8.4, 100 mM NaCl, 6 mM $MgCl_2$ and 6 mM β-mercaptoethanol at 65°C for 20 minutes with 4 units of Taq I. After incubation the reaction mixture was extracted with phenol, precipitated with ethanol, dissolved in water and electrophoresed on a 5% polyacrylamide gel. After electrophoresis the gel was stained with ethidium bromide and the 234 bp DNA band excised from the gel and the DNA recovered (A M Maxam and W Gilbert, Proc Natl Acad Sci USA 74, 560-564, 1977).

This DNA fragment was then inserted into the Cla I site of the plasmid pAT153 (Twigg and Sherratt, Nature 283, 216, 1980). 10 µg of pAT153 was digested to completion with Cla I and the 5'-phosphate groups removed by incubation at 37°C for 1 hour with 0.5 units of calf intestine alkaline phosphatase in 10 mM Tris-HCl, pH 8. This procedure prevents the plasmid recircularising in the absence of an inserted DNA fragment. 100 ng of the above treated pAT153 was ligated at 15°C for 4 hours to 5 ng of the 234 bp DNA in a reaction containing 50 mM Tris-HCl pH 7.6, 10 mM $MgCl_2$, 20 mM dithiothreitol, 1 mM ATP and 20 units of T4 DNA ligase. The ligated DNA was then used to transform competent E coli K-12 strain HB101 (Boyer, H W and Roulland-Dussoix, D J Mol Biol 41, 459-472, 1969) by standard techniques (Hershfield, V et al Proc Natl Acad Sci USA 71, 3455-3459, 1974) and the bacteria plated on L-agar plates containing 100 µg/ml ampicillin. Several ampicillin resistant colonies were selected, plasmid DNA prepared and the presence of the 234 bp fragment confirmed by restriction analysis.

The resulting plasmid, designated pCT12, has the structure shown in Fig .8. It should be pointed out that insertion of the Taq fragment into the Cla I site reforms a Cla I site at the end downstream from the trp promoter.

## MODIFICATION OF pCT12

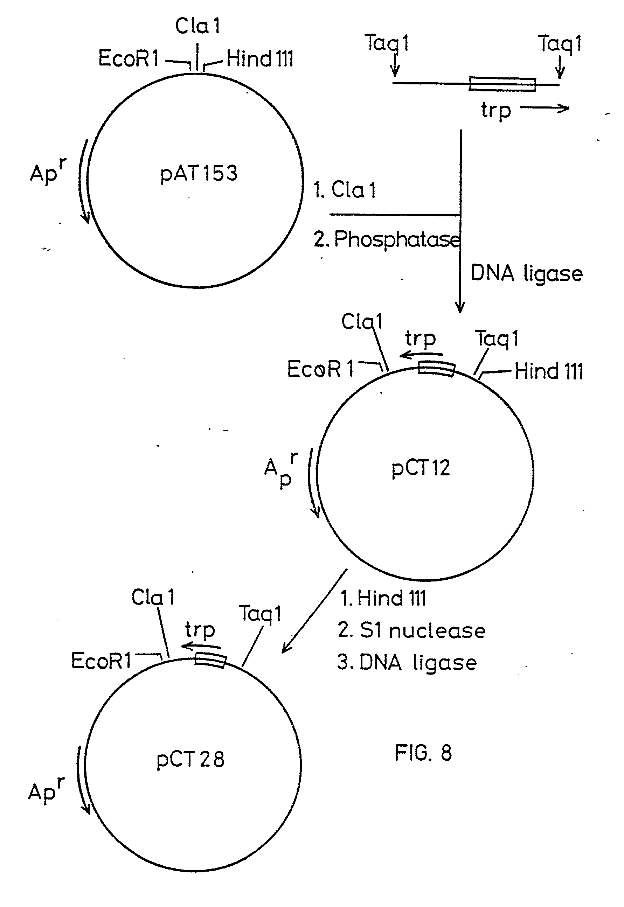

pCT12 was further modified to produce pCT28 and pCT29 as follows. 2 µg of pCT12 was digested to completion with Hind III and then incubated at 20°C for 30 minutes with 60 units of SI nuclease in a buffer containing 25 mM sodium acetate, pH 4.5, 0.3 M NaCl and 1 mM zinc acetate to remove the protruding Hind III ends. The reaction was terminated by raising the pH to 7.6 and extracting the mixture with phenol/chloroform (1 : 1). The DNA was concentrated by ethanol precipitation and a sample incubated with T4 DNA ligase as described above and then used to transform E coli K12 strain HB101. Several ampicillin resistant clones were selected, plasmid DNA prepared and the absence of the Hind III site confirmed by restriction analysis. The resulting plasmid, designated pCT28, has the structure shown in Fig 8.

pCT28 was further modified. 2 µg of EcoRI-digested pCT28 was incubated with 4 units of E coli DNA polymerase for 15 minutes at 10°C in a reaction containing 50 mM Tris-HCl pH 7.6, 10 mM $MgCl_2$,

10 mM β-mercaptoethanol, 0.2 mM dATP and 0.2 mM TTP. After incubation the mixture was extracted with phenol, with chloroform and then ethanol precipitated. This treatment causes the 4 nucleotides complementary to the 5' protruding ends of the EcoRI site to be filled in:-

$$5' \quad A\ A\ T\ T\ C\ - \qquad 5' \quad A\ A\ T\ T\ C\ -$$
$$3' \qquad\qquad G\ - \quad\longrightarrow\quad 3' \quad T\ T\ A\ A\ G\ -$$

0.6 µg of the above treated pCT28 was treated with 50 units of T4 DNA ligase in the presence of 30 picomoles of the 5'-phosphorylated synthetic oligonucleotide pCCAAGCTTGG and in 10 µl T4 DNA ligase buffer at 25°C for 16 hours. The mixture was then heated at 70°C for 10 minutes to stop the reaction and the linkers cleaved by digestion with Hind III. The linear DNA, now with Hind III ends, was separated from the linkers by electrophoresis on an agarose gel from which it was subsequently recovered by electroelution and concentrated by ethanol precipitation. Ligation, followed by transformation of E coli K-12 strain HB101, isolation of plasmid DNA and identification of plasmids with Hind III and EcoRI sites, produced the plasmid designated pCT29.

PREPARATION OF pCT29 FOR CLONING TRP E GENE

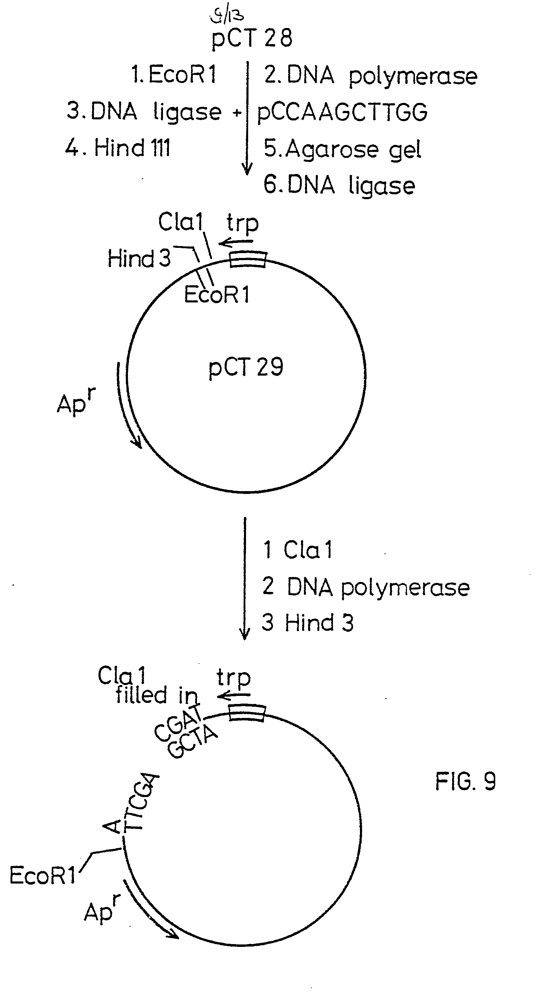

pCT29, Fig 9, contains the E Coli tryptophan promoter-operator region and, as well, unique restriction sites for the enzymes Cla I, Hind III and EcoRI downstream of the promoter region. Two of these sites, the Cla I and Hind III sites, were used to insert the trp E gene.

5 µg of pCT29 was cleaved with Cla I and the 5'-protruding ends filled in as described above using E coli DNA polymerase in the presence of dCTP and dGTP. The polymerase reaction mixture was extracted with phenol, then with chloroform and the DNA finally precipitated with ethanol. The filled-in DNA was then digested with Hind III and the resulting fragments separated by agarose gel electrophoresis. The largest fragment was isolated from the gel by first staining with ethidium bromide, locating the DNA with ultraviolet light and cutting from the gel the portion of interest. The DNA was recovered from the gel fragment by electroelution and concentrated by ethanol precipitation.

ISOLATION OF THE TRP E GENE

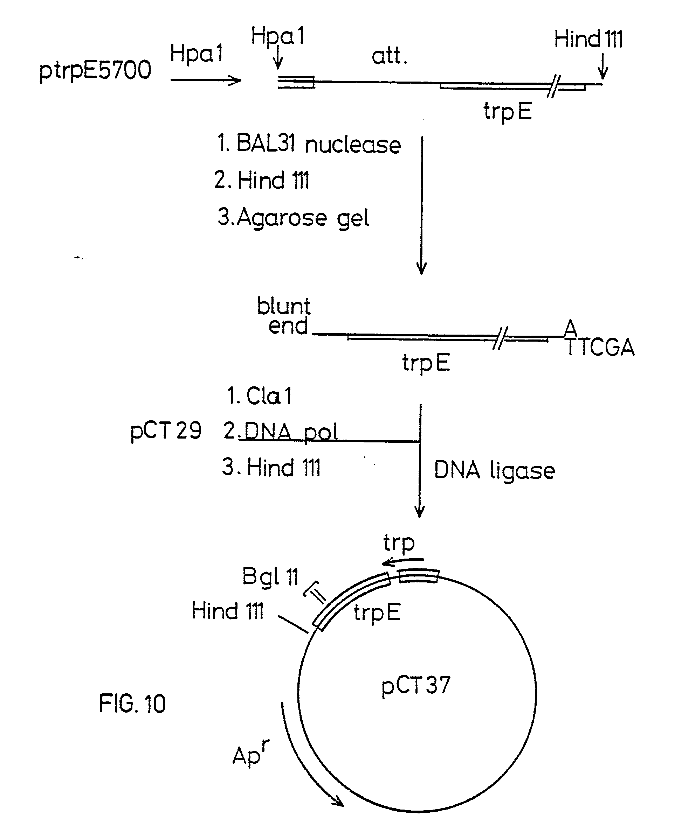

The trp E gene is present on the plasmid ptrp $E_{5700}$, a portion of which is shown in Fig 10. As described above however, ptrp $E_{5700}$ also contains the trp attenuator, a region concerned in the control of transcription from the trp attenuator as follows. To remove the/ attenuator region 5 $\mu$g of ptrp $E_{5700}$ was digested with Hpa I and then treated with nuclease BAL 31. This nuclease is a highly specific nuclease that can be used to shorten DNA fragments from the ends. Further, its action produces blunt-ended molecules. 5 $\mu$g of Hpa I digested ptrp $E_{5700}$ was treated with 1.5 units of BAL 31 nuclease in 0.6 M NaCl, 12 mM $CaCl_2$, 12 mM $MgCl_2$, 20 mM Tris-HCl pH 8, 1 mM EDTA at 30°C for 1.5 minutes. The mixture was then phenol extracted, chloroform extracted and ethanol precipitated. This treatment removes 150-250 bp from each end of the DNA fragments and so will remove the attenuator region from most molecules as it is 150 bp from the Hpa I site. The above treated DNA fragments were digested to completion with Hind III and then separated by agarose gel electrophoresis. DNA containing the trp E gene was isolated from the gel by first staining the gel with ethidium bromide, locating the DNA with ultraviolet light and cutting from the gel DNA in the size range 1800-1850 bp. This DNA was recovered from the gel fragment by electroelution and concentrated by ethanol precipitation.

## INSERTION OF THE TRP E GENE INTO pCT29

The trp E fragments isolated above can be inserted into pCT29 that had been modified as described above. Fig 10 illustrates the ligation reaction. 0.2 μg of modified pCT29 and 80 ng of the trp E fragment were incubated at 20°C for 16 hours with 100 units T4 DNA ligase in 50 mM Tris pH 7.6, 10 mM $MgCl_2$, 1 mM ATP and 20 mM dithiothreitol. The mixture was then used to transform E coli K12 strain HB101 as described above and transformants selected on L-agar plates containing ampicillin. Several ampicillin resistant colonies were selected, plasmid DNA isolated and examined by restriction analysis. This analysis confirmed the presence of the trp E gene and the loss of the alternator region. One of the plasmids with these characteristics, designated pCT37, was selected for further work involving the human calcitonin gene.

## CALCITONIN AND CALCITONIN-GLY FUSIONS WITH TRP E

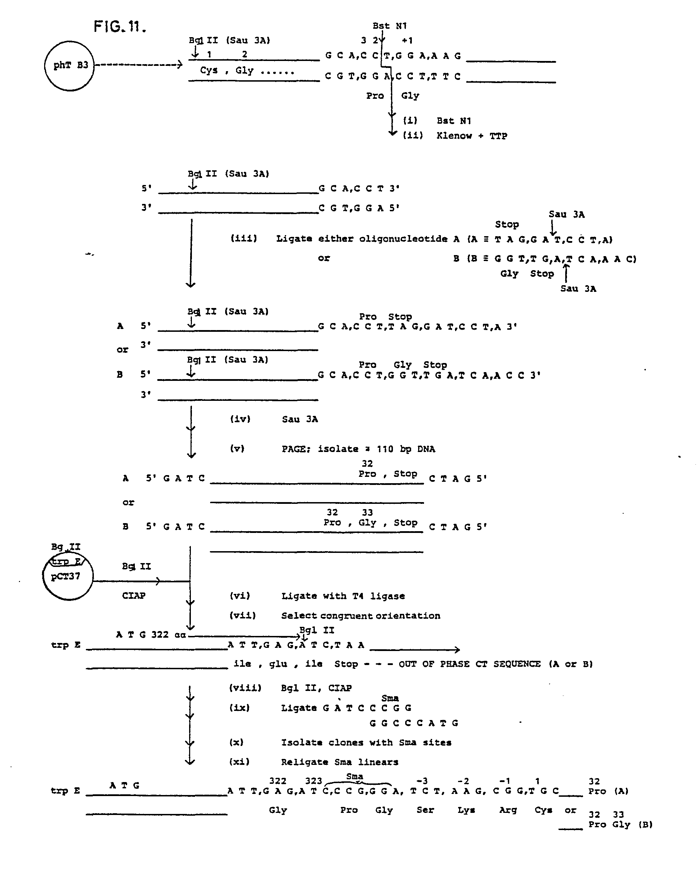

The scheme outlined in Fig 11 shows the steps carried out to combine the human calcitonin sequence derived from plasmid phT-B3 with the expression vector pCT37 described above to produce fusion proteins. The initial step involves the modification of the calcitonin sequence so that the ultimate amino acid in the fusion protein is either the proline corresponding to the authentic terminal amino acid in calcitonin or so that a further glycine is translated. The purpose of this construction relates to the final processing envisaged in calcitonin production and is described fully below. 20 μg of phT-B3 were incubated in 6 mM Tris-HCl pH 7.5, 6 mM MgCl$_2$, 6 mM β-mercaptoethanol and 20 mM KCl with 10 units of Bst NI for 60 minutes at 60°C. After incubation the reaction was made 0.3 M in NaAcetate pH 6.0, extracted with phenol, then chloroform and concentrated by ethanol precipitation. The precipitated DNA was washed with 70% ethanol, dried under vacuum and redissolved in 20 μl of water (Step i Fig 11. Bst NI cuts the calcitonin sequence only in the proline at position 32 and in such a way that the T residue in the anti-coding strand is removed. This T residue is added back in the next step (ii). 10 μg of the Bst NI cleaved phT-B3 were incubated in 50 mM Tris-HCl pH 7.6, 10 mM MgCl$_2$, 10 mM β-mercaptoethanol, 0.2 mM dCTP, 0.2 mM dTTP and 6 units of Klenow enzyme (DNA-polymerase large fragment; supplied by the Boehringer Corporation (London) Ltd). After the incubation the DNA was phenol extracted, chloroform extracted, ethanol precipitated, washed in 70% ethanol and redissolved in 10 μl of water. The resulting DNA is now flush-ended and can be ligated to other flush-ended DNA molecules. The DNA was ligated (covalently joined) with two separate synthetic oligonucleotides designated A or B in Fig 11.

A = T A G G A T C C T A
    A T C C T A G G A T

B = G G T T G A T C A A C C
    C C A A C T A G T T G G

Thus 2.5 μg of the phT-B3 derived DNA fragments were incubated in separate reactions with 400 ng of oligonucleotides A or B in 60 mM Tris-HCl pH 7.5, 8 mM MgCl$_2$, 10 mM β-mercaptoethanol, 1 mM ATP and 1.5 μl T4 DNA ligase (New England Biolabs Lot 17) for 24 hours at 16°C. Ligase activity was destroyed by raising the temperature to 70°C for 5 minutes and the DNA ethanol precipitated, washed in 70% ethanol, dried under vacuum and redissolved in 10 μl of water. 1 μl of both DNA samples (ie ligated with oligonucleotide A or B) was analysed for efficient ligation, as evidenced by its increase in size through concatemerisation and visualised by staining an agarose gel with ethidium bromide following an electrophoretic size separation. Note that the two ligation reaction samples represent the start of two separate and parallel construction routes (ie A or B) as shown in Fig11. The description of the experiments below apply for both series of constructions. The remaining plasmid DNA (9 μl) was separated from unreacted oligonucleotides by Sephadex G-50 chromatography and concentrated by ethanol precipitation. The DNA was redissolved in 50 μl 6 mM Tris-HCl, 50 mM NaCl, 6 mM $MgCl_2$, 6 mM β-mercaptoethanol and excess Sau 3A enzyme added to ensure complete cutting at all Sau 3 sites after 1 hour at 37°C.

The linker oligonucleotides contain the Sau 3A recognition sequence 5' GATC 3' and the Bgl II site 5' AGATCT 3' is also a Sau 3A site so that after the Sau 3A digestion, the calcitonin sequence resides in DNA fragments about 110 base pairs long (the exact length differing by one nucleotide depending on whether oligonucleotide A or B was used). These fragments were isolated following polyacrylamide gel electrophoresis (PAGE) as described above (Step iv Fig11) and following ethanol precipitation, were dissolved in water (10 μl).

The vector expression plasmid pCT37 (described in detail above) was prepared for their ligation into the Bgl II site as follows. 10 μg of pCT37 was incubated in 6 mM Tris-HCl, 50 mM NaCl, 6 mM $MgCl_2$, 6 mM β-mercaptoethanol and 10 units Bgl II for 60 minutes at 37°C. The linearised DNA was diluted to 0.5 ml and made 0.3 M in sodium acetate pH 6.0. 5' Phosphate groups were removed by the addition of 3 units of calf intestinal alkaline phosphatase (CIAP) and incubation at 60°C for thirty minutes, after which a further 3 units of CIAP was added and a further 30 minute incubation carried out.

Phosphatase activity was destroyed by one phenol, two phenol-chloroform and three chloroform extractions, after which the DNA was ethanol precipitated and redissolved in water (10 µl). In Step vi the calcitonin containing fragments were ligated into the Bgl II site of pCT37 as follows. 0.1 µg of the Bgl II linearised and CIAP treated pCT37 was incubated with approximately 2 ng of the calcitonin containing fragments in 60 mM Tris-HCl pH 7.5, 8 mM $MgCl_2$, 10 mM β-mercaptoethanol, 1 mM ATP and 0.2 µl T4 DNA ligase (New England Biolabs Lot 17) for 16 hours at 16°C. The DNA was then used to transform frozen competent E coli HB101 using standard methods (cf Methods in Enzymology Vol 68, pp 326-331) and transformants resistant to ampicillin selected on L-agar plates containing 100 µg/ml ampicillin. Clones containing plasmids with the calcitonin sequence in the Bgl II site and in the correct orientation were identified and the constructions confirmed by DNA sequencing using the method of Maxam, A and Gilbert, W Proc Natl Acad Sci 74, 560 (1977). At this stage the calcitonin DNA translation reading frame is out of phase with the trp E gene reading frame and if this reading frame is being used by a ribosome, a stop codon (TAA) will be encountered after amino acid 322 of the trp E gene. Thus in contrast to the parent expression vector pCT37 which directs the overproduction of the trp E gene product, these constructions would be expected to direct the overproduction of a novel truncated protein 323 amino acids long (ie about 35K Daltons M Wt).

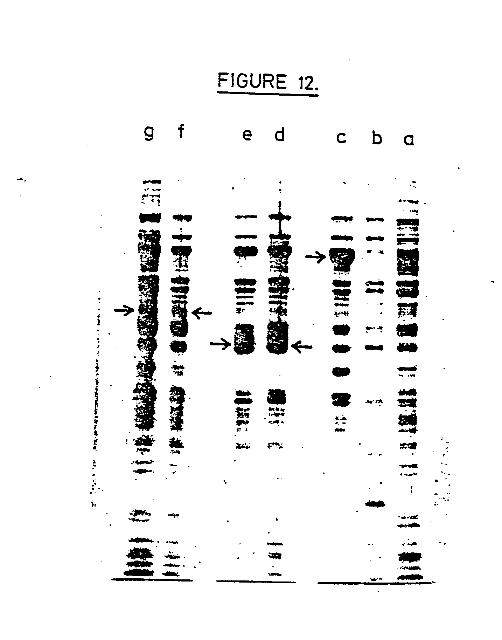

That such a novel protein is indeed produced by E coli harbouring these plasmids, and only under appropriate inducing conditions, is (see below). illustrated in Fig 12/ Thus the final steps in the construction of plasmids directing the overproduction of the desired trp E-calcitonin (or calcitonin-gly) fusion proteins requires the calcitonin sequence to be brought into the same translation reading frame as the trp E gene. This can be achieved by adding 3n + 2 nucleotides at the fusion junction between the trp E sequence and the calcitonin sequence (ie at the recreated unique Bgl II site). The synthetic octomer

$$5' \; G \; A \; T \; C \; C \; C \; G \; G$$

$$G \; G \; C \; C \; C \; T \; A \; G \text{ is suitable since it}$$

is of the correct length $(8 = 3 \times 2 + 2)$, has an appropriate 5' overhang for ligation into the Bgl II site and in addition creates a new unique restriction site for the enzyme Sma (5' CCCGGG 3') at the fusion junction. Thus the plasmids (10 µg) were incubated in 6 mM Tris-HCl, 50 mM NaCl, 6 mM β-mercaptoethanol, 6 mM $MgCl_2$ with 10 units Bgl II and the phosphate groups removed from the linear molecules using CIAP as described above. Following phenol extraction and ethanol precipitation 0.1 µg of the linear DNA was incubated with a 10-fold molar excess of the synthetic oligonucleotide

$$G \; A \; T \; C \; C \; C \; G \; G$$

$$G \; G \; C \; C \; T \; A \; G \text{ in 60 mM Tris-HCl pH 7.5,}$$

8 mM $MgCl_2$, 10 mM β-mercaptoethanol, 1 mM ATP and 0.2 µl T4 DNA ligase, for 16 hours at 16°C (step ix Fig 11). The ligation products were used to transform E coli HB101 cells and ampicillin resistant transformed cloned isolated on L-agar plates containing 100 µg/ml ampicillin.

The majority of plasmids in these clones were found to contain more than one copy of the synthetic oligonucleotide at the fusion junction. 1 µg of plasmids with 2 inserted linkers was linearised by incubation

in 6 mM Tris-HCl, 6mM MgCl$_2$, 6mM $\beta$-mercaptoethanol and 20mM KCl with 10 units of Sma I (supplied by the Boehringer Corporation (London) Ltd), and following purification of the linear molecules from unlinearised plasmid, the DNA was religated with T4 DNA ligase and following a further round of transformation into HB101 plasmids with the structure shown at the bottom of Fig.11. were isolated. As illustrated the DNA junction is now such that the calcitonin sequence is in phase with the trp E sequence and such plasmids showed therefore direct the production of a novel fusion protein of the expected size (about 38K Daltons M Wt, see Fig.12). Verification of these constructions has been demonstrated in the following manner. Thus E.Coli cells harbouring either no plasmids (i.e. HB101 cells described above, Fig.12 lane a) or harbouring pcT37 (lanes b and c) or harbouring the constructions described above in which a stop codon is found after 323 amino acids (lanes d and e) were grown at 37°C either in L-broth (lanes a and b) in which the trp promotor is not induced, or in M9-salts in which it is (lanes c,d,e,f,g - see also R.A.Hallewell and J.S.Emtage, Gene 9, 27-47, 1980) and harvested by centrifugation. The cells were dissolved in SDS sample buffer (Laemmli, U.K.,Nature 227, 680 (1970). Protein equivalent to 50 µl of original culture was electrophoresed on a 12.5% (w/v) polyacrylamide gel and the separated proteins visualized by staining with coomassie blue. It is clear from Fig.12. that pcT37 under conditions of Trp promotor induction overproduces the Trp E gene (lane c, indicated by arrow - cf. lane b, where growth is under non-induced conditions). In contrast, in its out of phase calcitonin containing derivatives (lane d has the A type end defined in Fig.11. while e has the B type end) an appropriate sized novel protein is present as indicated. Where the two constructions contain the calcitonin sequence in phase (lane f in the A-type and lane g the B-type construction) novel proteins of the expected size can be seen.

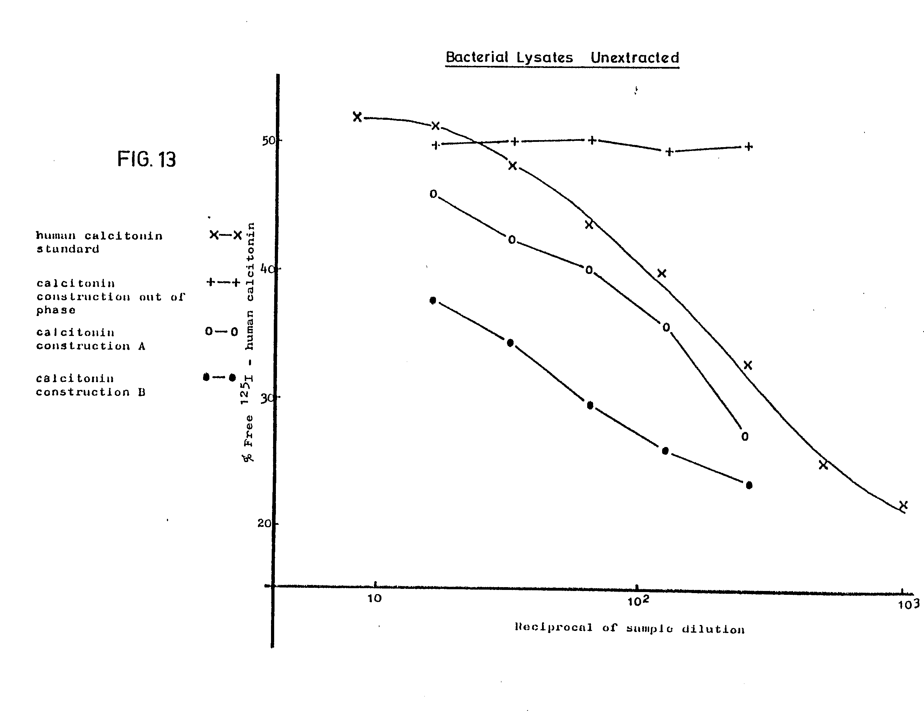

Identification of the presence of the calcitonin peptide with bacterial cell lysates was performed by radioimmunoassay. The assay was carried out essentially as described by SI Girgis et al., (J.Endocrinol 78, 372-382). As can be seen in Figure 13, dilutions from a lysate of an E.coli carrying the calcitonin gene inserted out of phase failed to compete out the $^{125}$I-labelled human calcitonin. In contrast lysates obtained from E.coli carrying constructions of the calcitonin gene (both A and B) inserted in phase competed with $^{125}$I-labelled human calcitonin in the expected manner (for comparison compare with calcitonin standard). This confirms that the constructions resulted in the synthesis of a fusion protein containing the expected antigenic determinants of human calcitonin.

## CALCITONIN FROM A TRP E-CALCITONIN FUSION PROTEIN

The ultimate aim of introducing the human calcitonin sequence into the trp E gene of E.coli in such a way that substantial quantities of a trp E- calcitonin fusion protein are produced is to liberate the calcitonin peptide in a commercially viable way. This may be done by several possible routes. As a first step the calcitonin peptide must be cleaved from the fusion protein. This can be achieved by the use of the enzyme trypsin which will cleave exactly at the fusion junction since the initial cysteine residue is preceded by an arginine. Calcitonin contains no arginines but one lysine residue at position 18 which would also be liable to trypsin cleavage. This lysine can however be protected by citraconic anhydride (Shine S, Fettes I, Nancy C.Y.Lan, Roberts,J.L.and Baxter,J.D.

Nature 285, 456-461). The peptide liberated by this procedure differs from authentic calcitonin only in that in authentic calcitonin the C-terminal amino acid is a prolinamide rather than a proline (or proline-glycine) amino acid. Conversion of the liberated peptide into authentic calcitonin is possible through the use of C-terminal modification activity of yeast carboxypeptidase Y (Breddam, K, Widmer, F and Johanson, J.T.Carlsberg Res. Commun. 45, 237-247 and 361-367 1980).

Quite apart from the biological use of calcitonin produced in accordance with the invention, the availability of a cloned human calcitonin cDNA probe, also made possible by the invention, permits comparisons to be drawn between human calcitonin gene structure and expression in normal thyroid tissue, and in familial and sporadic medullary carcinoma of the thyroid. The probes may also be used for definitive studies of the molecular mechanisms involved in the ectopic synthesis of calcitonin, in particular by lung carcinoma, whilst the possibility also arises that cDNA probes may be used to investigate linked DNA polymorphisms, thereby providing a quick and reliable means of distinguishing between familial and sporadic medullary carcinoma of the thyroid, saving patients and their relatives from uncomfortable, extensive and often invasive investigations.

- 32 -

CLAIMS

1. A structural gene encoding a polypeptide comprising the amino acid sequence of human calcitonin, in which said polypeptide is processable to produce human calcitonin.

2. A structrual gene according to claim 1, in which the polypeptide is a fusion protein comprising a host protein and a peptide comprising the amino acid sequence of human calcitonin.

3. A structural gene according to claim 1, in which the polypeptide comprises human calcitonin precursor polyprotein.

4. A DNA transfer vector containing a structural gene according to any of claims 1-3.

5. A DNA transfer vector according to claim 4 having an inserted (poly)nucleotide fragment encoding a polypeptide including the following amino acid sequence:-

-cys-gly-asn-leu-ser-thr-cys-met-leu-gly-thr-
-tyr-thr-gln-asp-phe-asn-lys-phe-his-thr-phe-
-pro-gln-thr-ala-ile-gly-val-gly-ala-pro-.

6. A DNA transfer vector according to claim 4, having an inserted (poly)nucleotide fragment encoding a polypeptide which includes the following amino acid sequence:-

-val-leu-leu-ala-ala-leu-val-gln-asp-tyr-val-gln-met-
-lys-ala-ser-glu-leu-glu-gln-glu-gln-glu-arg-glu-gly-
-ser-ser-leu-asp-ser-pro-arg-ser-lys-arg-
-cys-gly-asn-leu-ser-thr-cys-met-leu-gly-thr-
-tyr-thr-gln-asp-phe-asn-lys-phe-his-thr-phe-
-pro-gln-thr-ala-ile-gly-val-gly-ala-pro-
-gly-lys-lys-arg-asp-met-ser-ser-asp-leu-glu-arg-
-asp-his-arg-pro-his-val-ser-met-pro-gln-asn-ala-asn-.

7. A polypeptide containing the amino.acid sequence of human calcitonin which is processable to produce human calcitonin.

8. A polypeptide according to claim 7 consisting of a fusion protein comprising a host protein in combination with a peptide comprising the amino acid sequence of human calcitonin.

9. A polypeptide according to claim 7 comprising the amino acid sequence of human calcitonin precursor.

10. A method for the production of human calcitonin comprising

    (i) inserting a gene encoding a polypeptide comprising the amino acid sequence of human calcitonin in a DNA transfer vector;

    (ii) transforming a host organism with the gene-containing DNA transfer vector; and

    (iii) recovering polypeptide expressed by the transformed organism and processing said polypeptide to produce human calcitonin.

11. A method of forming a DNA transfer vector having a nucleotide sequence coding for a polypeptide comprising the amino acid sequence of human calcitonin, comprising

    (i) providing mRNA coding for a polypeptide comprising the amino acid sequence of human calcitonin;

    (ii) synthesizing a double stranded cDNA one strand of which has a nucleotide sequence complementary to that of the mRNA, and

    (iii) inserting said double stranded cDNA in a DNA transfer vector.

12. A method as claimed in claim 11 wherein the said mRNA is provided by cells containing said mRNA originating from the thyroid gland of a calcitonin producing organism.

13. A DNA transfer vector produced by the method claimed in claim 11 or claim 12.

14. A DNA transfer vector according to claim 4,5,6 or 13 transferred to and replicated in a microorganism strain.

15. A DNA transfer vector according to claim 14 wherein the microorganism is a bacterium and the transfer vector is a plasmid.

16. A DNA transfer vector according to claim 15 wherein the microorganism is a strain of <u>Escherichia</u> <u>coli</u>.

17. A method for the production of human calcitonin comprising processing a polypeptide to produce human calcitonin in which said polypeptide has been expressed by a host organism which has been transformed with a gene-containing DNA transfer vector comprising an inserted gene encoding a polypeptide comprising the amino acid sequence of human calcitonin.

FIGURE 1

A.                    i    ii    iii

mRNA                  −    +    +

36 000 ⟶

23 300 ⟶
21 000 ⤏

14 300 ⟶

Ab ppt        −    −    +

B.        i    ii    iii    iv    v    vi    vii    viii    C.  i    ii    iii    iv

mRNA      +    −    +    −    +    −    +    −        +    +    +    +
Mg²⁺ mM  1.5  1.5  2.0  2.0  2.25  2.25  2.5  2.5    1.5  1.5  2.5  2.5
Ab ppt.   −    −    −    −    −    −    −    −        −    +    −    +

36 000 ⟶

23 300 ⟶
21 000 ⤏

14 300 ⟶

FIG. 2.

A.

| bp | | bp |
|---|---|---|
| 910 | | |
| 659 | | 587 |
| 655 | | |
| 521 | | 458 |
| 403 | | 434 |
| | | 339 |
| 281 | | 267 |
| 257 | | 234 |
| 226 | | 213 |

B.

MW

36 000
23 300
21 000
14 300
11 700

C.

phT-B6

HaeIII  AluI  Sau 3A  SphI

Bst NI  RsaI  SphI  Bst NI  SphI

Hinf I  HaeIII  SacI

Bst NI  Hinf I  Sau 3A

phT-B3

100 bp

0070675

# FIGURE 3

i  ii  iii  iv  v

Origin ⟶

(linear pBR 322 DNA)   4362 ⟶

(Pst I/Bam HI pBR 322)  3237 ⟶

(18S-rRNA)  2060 ⟶

(Pst I/Bam HI pBR 322)  1125 ⟶

(E.coli tRNA)  80 ⟶

# FIGURE 4

```
       -36 -35 -34 -33 -32 -31 -30 -29 -28 -27 -26 -25 -24 -23 -22 -21 -20 -19 -18 -17 -16 -15 -14 -13 -12 -11 -10 -9
       Val Leu Leu Ala Ala Leu Val Gln Asp Tyr Val Gln Met Lys Ala Ser Glu Leu Glu Gln Glu Gln Glu Arg Glu Gly Ser Ser

   5' GTC CTG CTG GCT GCA CTG GTG CAG GAC TAT GTG CAG ATG AAG GCC AGT GAG CTG GAG CAG GAG CAA GAG AGA GAG GGC TCC AGC
   3' CAG GAC GAC CGA CGT GAC CAC GTC CTG ATA CAC GTC TAC TTC CGG TCA CTC GAC CTC GTC CTC GTT CTC TCT CTC CCG AGG TCG


   -8  -7  -6  -5  -4  -3  -2  -1   1   2   3   4   5   6   7   8   9  10  11  12  13  14  15  16  17  18  19  20  21  22  23
   Leu Asp Ser Pro Arg Ser Lys Arg Cys Gly Asn Leu Ser Thr Cys Met Leu Gly Thr Tyr Thr Gln Asp Phe Asn Lys Phe His Thr Phe Pro

   CTG GAC AGC CCC AGA TCT AAG CGG TGC GGT AAT CTG AGT ACT TGC ATG CTG GGC ACA TAC ACG CAG GAC TTC AAC AAG TTT CAC ACG TTC CCC
   GAC CTG TCG GGG TCT AGA TTC GCC ACG CCA TTA GAC TCA TGA ACG TAC GAC CCG TGT ATG TGC GTC CTG AAG TTG TTC AAA GTG TGC AAG GGG
                                        ←


   24  25  26  27  28  29  30  31  32   +1  +2  +3  +4   +5  +6  +7  +8  +9 +10 +11 +12 +13 +14 +15 +16 +17 +18 +19 +20 +21 +22
   Gln Thr Ala Ile Gly Val Gly Ala Pro Gly Lys Lys Arg Asp Met Ser Ser Asp Leu Glu Arg Asp His Arg Pro His Val Ser Met Pro Gln

   CAA ACT GCA ATT GGG GTT GGA GCA CCT GGA AAG AAA AGG GAT ATG TCC AGC GAC TTG GAG AGA GAC CAT CGC CCT CAT GTT AGC ATG CCC CAG
   GTT TGA CGT TAA CCC CAA CCT CGT GGA CCT TTC TTT TCC CTA TAC AGG TCG CTG AAC CTC TCT CTG GTA GCG GGA GTA CAA TCG TAC GGG GTC
                                                      →


   +23 +24 +25
   Asn Ala Asn term

   AAT GCC AAC TAA ACTCCTCCCTTTCCTTCCTAATTTCCCTTCTTGCATCCTTCCTATAACTTGATGCATGTGGTTTGGTTCCTCTCTGGTGGCTCTTTGGGCTGGTATTGGTGGCTTTC
   TTA CGG TTG ATT TGAGGAGGGAAAGGAAGGATTAAAGGGAAGAACGTAGGAAGGATATTGAACTACGTACACCAAACCAAGGAGAGACCACCGAGAAACCCGACCATAACCACCGAAAG


   CTTGTGGCAGAGGATGTCTCAAACTTCAAGATGGGAGGAAAGAGAGCAGGACTCACAGGTTCGGAAGAGAATCACCTGGGAAAATACCAGAAAATGAGGGCCGCTTTGAGTCCCCCAGAGATGT
   GAACACCGTCTCCTACAGAGTTTGAAGTTCTACCGTCCTTTCTCTCGTCCTGAGTGTCCAACGTTCTCTTAGTGGACCCTTTTATGGTCTTTTACTCCCGGCGAAACTCACGGGGTCTCTACA


   CATCAGAGCTCCTCTGTCCTGCTTCTGAATGTGCTGATCATTTGAGC AATAAA ATTATTTTTCCCC(A)n 3'
   GTAGTCTCGAGGAGACAGGACCGAAGACTTACACGACTAGTAAACTCC TTATTT TAATAAAAAGGGG(T)n 5'
```

G   G+A   A>C   C   C+T

FIG. 5.

FIG. 6.

FIG. 7.

0070675

8/13

FIG. 8

9/13

pCT 28

1. EcoR1 | 2. DNA polymerase

3. DNA ligase + | pCCAAGCTTGG

4. Hind 111 | 5. Agarose gel

6. DNA ligase

Cla1  trp

Hind 3

EcoR1

pCT 29

Ap$^r$

1 Cla 1

2 DNA polymerase

3 Hind 3

Cla 1
filled in  trp

CGAT
GCTA

TTCGA

A

EcoR1

Ap$^r$

FIG. 9

FIG. 10

11/13

FIG. 11.

phT B3 - - - - - - →

Bgl II (Sau 3A)
↓ 1    2 _____
Cys , Gly ...... _____

Bst N1
3 2↓ +1
G C A,C C T,G G A,A A G _____
C G T,G G A,C C T,T T C _____
Pro | Gly

↓ (i)   Bst N1
→ (ii)  Klenow + TTP

Bgl II (Sau 3A)
5' ↓ _____ G C A,C C T 3'
3' _____ C G T,G G A 5'

(iii)   Ligate either oligonucleotide A (A ≡ T A G,G A T,C C T,A)
or
B (B ≡ G G T,T G,A,T C A,A A C)
Gly  Stop ↑
Sau 3A

Stop        Sau 3A
↓

Bgl II (Sau 3A)
A  5' ↓ _____ Pro  Stop
G C A,C C T,T A G,G A T,C C T,A 3'
or  3' _____

Bgl II (Sau 3A)
B  5' ↓ _____ Pro  Gly  Stop
G C A,C C T,G G T,T G A,T C A,A C C 3'
3' _____

(iv)   Sau 3A

(v)    PAGE; isolate ≈ 110 bp DNA

32
A  5' G A T C _____ Pro , Stop  C T A G 5'

or

32   33
B  5' G A T C _____ Pro , Gly , Stop  C T A G 5'

Bgl II
trp E/
pCT37
Bgl II

CIAP

(vi)   Ligate with T4 ligase

(vii)  Select congruent orientation

A T G 322 aa _____ Bgl II
trp E _____ A T T,G A G,A T C,T A A _____→
_____ ile , glu , ile Stop - - - OUT OF PHASE CT SEQUENCE (A or B)

(viii) Bgl II, CIAP
Sma
(ix)   Ligate G A T C C C G G
G G C C C A T G

(x)    Isolate clones with Sma sites

(xi)   Religate Sma linears

Sma
trp E  A T G _____ 322  323 ⌢ -3   -2   -1    1        32
A T T,G A G,A T C,C C G,G G A, T C T, A A G, C G G,T G C ____ Pro (A)
_____ Gly        Pro  Gly  Ser  Lys  Arg  Cys  or  32  33
____ Pro Gly (B)

## FIGURE 12.

Bacterial Lysates  Unextracted

FIG. 13

human calcitonin standard    x—x

calcitonin construction out of phase    +—+

calcitonin construction A    0—0

calcitonin construction B    ●—●

% Free $^{125}$I – human calcitonin

Reciprocal of sample dilution

13/13

0070675

European Patent
Office

EUROPEAN SEARCH REPORT

0070675
Application number

EP 82 30 3667

## DOCUMENTS CONSIDERED TO BE RELEVANT

| Category | Citation of document with indication. where appropriate, of relevant passages | Relevant to claim | CLASSIFICATION OF THE APPLICATION (Int. Cl. ³) |
|---|---|---|---|
| Y | PROC. NATL. ACAD. SCI., vol.77, no.8, August 1980, (US) S.G. AMARA et al.: "Characterization of rat calcitonin mRNA", pages 4444-4448 * the whole article * | 1-4,11 -16 | C 12 N 15/00 C 12 P 21/02 C 07 C 103/52 |
| X | CHEMICAL ABSTRACTS, vol.93, no.17, October 27, 1980, page 218, abstract no.162892q, Columbus, Ohio (US) C. DESPLAN et al.: "Cell free translation of mRNA coding for human and murine calcitonin" & FEBS Lett. 1980, 117(1), 89-92 * the whole abstract * | 7 | |
| Y | CHEMICAL ABSTRACTS, — | 1-4,11 -16 | TECHNICAL FIELDS SEARCHED (Int. Cl. ³) |
| X | CHEMICAL ABSTRACTS, vol.92, no.7, February 18, 1979, page 216, abstract no.53546p, Columbus, Ohio (US) R.H. GOODMAN et al.: "Cell-free translation of messenger RNA coding for a precursor of human calcitonin" & Biochem. Biophys. Res. Commun. 1979, 91(3), 932-8 * the whole abstract * | 7 | C 12 N C 12 P |
| Y | CHEMICAL ABSTRACTS, | 1-4,11 -16 | |

-/-

The present search report has been drawn up for all claims

| Place of search | Date of completion of the search | Examiner |
|---|---|---|
| THE HAGUE | 06-10-1982 | DESCAMPS J.A. |

European Patent
Office

**EUROPEAN SEARCH REPORT**

| | **DOCUMENTS CONSIDERED TO BE RELEVANT** | | | Page  2 |
|---|---|---|---|---|
| Category | Citation of document with indication. where appropriate, of relevant passages | Relevant to claim | | CLASSIFICATION OF THE APPLICATION (Int. Cl. ³) |
| P,X | BIOCHEM. J., vol.199, 1981, (GB)<br><br>J. ALLISON et al.: "The con-struction and partial characterization of plasmids con-taining complementary DNA sequences to human calcitonin precursor polyprotein", pages 725-731 * the whole article *<br><br>--- | 1-4,7-9,11-16 | | |
| P,X | CHEMICAL ABSTRACTS, vol.95, no.15, October 12, 1981, page 199, abstract no.127640a, Columbus, Ohio (US)<br>J.W. JACOBS et al.: "Calcitonin messenger RNA encodes multiple polypeptides in a single precur-sor. & Science (Washington, D.C., 1883) 1981, 213(4506), 457-9 * the whole abstract *<br><br>----- | 1-4 | | TECHNICAL FIELDS SEARCHED (Int. Cl. ³) |

The present search report has been drawn up for all claims

| Place of search | Date of completion of the search | Examiner |
|---|---|---|
| THE HAGUE | 06-10-1982 | DESCAMPS J.A. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO Form 1503 03 82